# EUROPEAN PATENT SPECIFICATION

(11) **EP 2 544 601 B1**
(45) Date of publication and mention of the grant of the patent: **27.06.2018**
(21) Application number: 11700940.7
(22) Date of filing: 21.01.2011
(51) Int. Cl.: A61B 17/17

(54) **ORTHOPAEDIC INSTRUMENT**
ORTHOPÄDISCHES INSTRUMENT
INSTRUMENT ORTHOPÉDIQUE

(30) Priority: 10.03.2010 GB 201003921
(43) Date of publication of application: 16.01.2013
(73) Proprietor: Johnson & Johnson Medical GmbH, 22851 Norderstedt (DE)
(72) Inventor: MANNSS, Jurgen, 81825 Munchen (DE); BURGER, Thorsten, 80804 Munchen (DE)
(74) Representative: Alton, Andrew
(86) International application number: PCT/EP2011/050862
(87) International publication number: WO 2011/110374

(56) References cited:
- EP-A1- 0 591 985
- EP-A1- 1 457 159
- US-A1- 2008 243 127
- US-A1- 2009 163 922

## Description

Orthopaedic procedures often involve the use of prosthetic implants to repair or replace joint parts. Various tools and instruments are used in the surgical procedure to prepare the joint to receive the implant components and also to place and implant the prosthetic implant. As part of the procedure, it can be important to try and ensure that the implant is placed at an intended position and orientation so as to try and achieve the intended effect of the procedure. Hence, various instruments and guides are often used during an orthopaedic procedure to try and help accurately place and orient the implant.

Computer assisted surgery (CAS) systems can be used to help plan an implant position and navigate placement of the implant. However, such systems are expensive and not always available. Also, some surgeon's are resistant to using CAS systems for various reasons, for example because of personal surgical preferences or because the surgeon has to learn how to use the CAS system.

Orthopaedic procedures are often carried out on a number of joints in the body, such as knee joints and there is a well developed art in carrying out knee procedures. Hip and shoulder injuries are also an area in which orthopaedic procedures are often carried out. However, procedures carried out on ball and socket type joints often have their own particular difficulties which differ to the difficulties arising during knee procedures.

For example, in some hip procedures an important step can be the correct placing of a prosthetic acetabular cup component in the acetabulum of the patient's pelvis. The depth and/or orientation of implantation of the cup component can be important in the success of the hip procedure. It can be difficult to correctly place the acetabular component from images of the acetabulum as some of the parts of the hip joint are soft tissues, e.g. the labrum and the transverse acetabular ligament, which are often removed during preparation of the acetabulum. Hence, there can be no appropriate or easily accessible anatomical landmarks of the patient which can be used to guide the placement of the acetabular cup.

As another example, an important step in shoulder arthroplasty is the correct positioning of the total shoulder prostheses components and the associated bone preparation steps. The glenoid component position, its orientation and its size are important factors in determining the outcome of the procedure and incorrect positioning of the bone preparation instruments can reduce the post-operative range of motion of the patients's shoulder. In particular surgeons often have difficulties determining the appropriate positioning of bone preparation instruments for a pathologically deformed Glenoid bone structure when performing bone preparation in-situ to try and meet their pre-operative surgical plan.

Surgeons can perform surgical planning based on three dimensional CT image data of the patient's anatomy to define the implant size and location. The CT image data can also be used to provide an accurate model of the anatomy of the patient to help determine the required bone preparation steps. To estimate the required version angle corrections for a deformed Glenoid osteoarthritis bone, only one specific transverse CT slice is commonly used for this planning.

The guidance of surgical tools used for surface reaming, peg hole orientations and trajectories for Metaglene locking screws on the Glenoid and Humerus, can be difficult to achieve in order to obtain a proper bone stock for appropriate implant components. Typically only a limited exposure of the bone structure to be operated on is available to the surgeons for their intervention and for them to recognize the instrument position and orientation in relation to the patient's anatomy.

Previously used instruments and templates to transfer surgical plan information in-situ, are not designed to fulfil their function in relation to a 3D image or stereotactic coordinate system at the same time. Templates have been used previously but are designed to present the Glenoid size and centre information only visually. The template position on bone provides no guidance or feedback on the planned position for the preparation instruments. Other instruments are designed to target only the entry point position of a k-wire and/or an eccentric drill bit and not also to use orientation information, for example relative to the Glenoid and Scapula plane.

Hence for arthroplasty operations on ball and socket type joints, there are significant difficulties in transferring planed implant position into the surgical field so as to allow orientation position to be provided for guidance of instruments used during the procedure. US 2009/163922 describes an acetabular guide having a first surface and a second surface opposite to the first surface, the first surface made to conform to an acetabular rim surface around an acetabulum of a patient in accordance with a three-dimensional image of the acetabulum of the patient. The acetabular guide includes an aperture having an inner surface oriented at selected anteversion and abduction angles relative to the first surface for guiding an acetabular implant into the acetabulum at the selected anteversion and abduction angles. The guide is attached to the acetabulum using fasteners passing through apertures in a flange exteding around the guide.

US 2008/243127 describes a patient specific instrument according to the preamble of claim 1. EP1457159 describes a drill guide for positioning a glenoid implant. A plate delimits a support surface that is designed to press against a scapula of a patient. An adjusting unit adjusts the direction of guiding of a component, e.g. a drill bush, with respect to the plate. The adjusting unit is adapted to be received and fixed in a transversal opening formed in the plate and adapted to emerge in the support surface.

EP0591985 describes a glenoid alignment guide for aligning a drill bit relative to the glenoid and the glenoid neck of a patient. The alignment guide comprises a retractor plate for displacing the posterior and superior aspect of the deltoid muscle, and a drill guide slidably movable along the retractor plate.

Hence, it would be beneficial to provide an instrument which can be used to improve an orthopaedic procedure carried out on a ball and socket type joint.

According to a first aspect of the present invention, there is provided a patient specific instrument as defined in claim 1.

As the attachment mechanism is configured to match the shape of at least two portions of the rim of the bony socket of the patient, and the components of the instrument can only be assembled and locked together to form the instrument when the attachment mechanism is correctly located and mounted on the bony socket, the instrument is automatically correctly placed relative to the socket. Otherwise, the instrument cannot be assembled owing to the interlocking nature of its parts and the patient anatomy specific shape of the attachment parts of the instrument. Further, the instrument stays assembled when mounted on the rim of the socket as the socket itself contributes to the structural stability of the instrument in its assembled state.

The socket or bony socket of the patient may be any anatomical concavity having sufficient rim portion or portions to which the instrument can be attached. For example the socket can be the glenoid or the acetabulum of the patient.

The locking mechanism can be provided wholly or at least partially by the shape of portions or parts of the constituent components of the instrument. That is, no ancillary or separate fixings, may be needed in order for the components of the instrument to be assembled and locked together. The locking or interlocking mechanism can be provided by the shape or shape of a part or parts of the instrument. The locking or interlocking mechanism or mechanisms may be entirely mechanical. Some or all of the components of the instrument can be attachable or connectable to other or others of the components in a single way.

The body can include a plurality of guide formations for guiding different components. Hence, the same instrument can be used to guide components used during different stages of the procedure without having to reconfigure or adjust the instrument.

The or each guide formation can be in the form of an aperture or a channel. The or each guide formation can be configured to receive in use various different components, tools, preparation instruments, fixings or instruments used during an arthroplasty procedure. For example, the or each guide formation can be configured to guide a k-wire, drill bit, bone preparation instrument, reamer, bur, screw, pin or plug.

The attachment mechanism can include at least three parts each of which can engage the rim of the socket, each part being shaped to match the shape of a respective portion of the rim of the socket. Three parts helps to stabilise the attachment of the instrument to the socket. Also, using three parts can reduced the size of the parts needed to ensure that the parts are uniquely attachable to their respective portions of the rim of the socket.

Each of the parts of the attachment mechanism can be shaped to match the shape of their respective portion of the socket in a direction around the periphery of the socket rim and/or in a direction generally transverse to the periphery of the socket rim.

The parts of the attachment mechanism can be separate parts. The parts of the attachment mechanism can be in the form of clips.

The parts of the attachment mechanism can be parts of the same structure. That is they can all be formed as integral parts of a larger structure.

The body can comprise a plurality of parts. A one of the plurality of parts of the body can have a complex shaped surface which can mate with a matching shaped surface on another of the parts of the plurality of parts of the body so that the body can only be assembled in a single unique configuration. The body can have a generally curved shape, such as circular, ellipsoidal, round or oval.

Each part of the body can include a respective portion of the attachment mechanism. That is a portion of the attachment mechanism can be provided as an integral part of the body part. Interlocking formations of the parts of the body can provide the locking mechanism. The interlocking formations can include a lug or lugs and a recess or recesses shaped to receive the lug or lugs.

The attachment mechanism can be provided as an entirely separate part or parts to the body or body parts.

The locking mechanism can be provided by interlocking formations on the body and the parts of the attachment mechanism. The or each interlocking formation can include a lug or lugs and a recess or recesses shaped to receive the lug or lugs.

The attachment mechanism can be partially or wholly a shape memory material. The shape memory material can transition between a first state in which the instrument can be presented to the socket and a second state in which the attachment mechanism attaches the instrument to the socket at the single pre-planned position. The shape memory material can have a shape matching the shape of a portion of the socket to which it is intended to attach in the second state. A part of the attachment mechanism not made of shape memory material can have a shape matching the shape of a portion of the socket to which it is intended to attach.

The attachment mechanism can be at least partially a shape memory material. The shape memory material can allow one or more or each of the parts of the attachment mechanism to transform to a shape to match the shape of a respective rim portion of the rim of the socket.

The body can include a frame part. The body can further include a core. The core can be receivable in the frame. The core can include the at least one guide formation. A further core can be provided which can be substituted for the core in the frame. The further core can include a further guide formation.

The locking mechanism can be provided by interlocking formations on the frame and the parts of the attachment mechanism. The interlocking formations can include a lug or lugs and a recess or recesses shaped to receive the lug or lugs.

The patient specific instrument can further comprise a limiter, or keying component, allowing the core to be received in the frame in a single position. The limiter can be in the form of a mechanical stop. The limiter can prevent rotation of the core relative to the frame. The limiter can be adjustable to allow the position of the core to be changed relative to the frame. The limiter can be removable from the frame.

The frame can be made wholly or at least partially from an elastic material. The parts of the attachment mechanism can be provided as parts of the frame. The frame can act to urge the parts of the attachment mechanism to engage about the rim of the socket when the instrument is assembled and attached to the glenoid.

A part of the instrument can include a socket facing surface and the socket facing surface is shaped to match the shape of a portion of the socket of the patient against which the socket facing surface will abut when the instrument is attached to the socket of the patient only at the single pre-planned position. The portion of the socket can be a part of the surface of the inner cavity of the socket or a portion of the socket adjacent the rim of the socket, and either toward the inner cavity of the socket or away from the inner cavity of the socket. The part of the instrument can be a part of or the whole of the body or a part of the body. The part of the instrument can be a part of or the whole of one, a plurality or all of the attachment parts or a portion of the attachment parts.

The patient specific instrument can be disassembled in situ. That is the instrument can be disassembled while attached to the bone of the patient so as to provide better access to the surgical site. The locking mechanism can be configured to allow the instrument to be disassembled. The locking mechanism can comprise entirely interlocking formations. According to a further aspect of the invention, there is provided a method for producing a patient specific instrument according to the first aspect of the invention, as defined by claim 16.

The ball and socket joint can be a shoulder or a hip. For example, a CT image of the patient's shoulder or hip can be captured. Planning software can be used to plan a position and/or orientation to be used during the surgical procedure. The position and/or orientation can be for a glenoid prosthetic component or an acetabular cup prosthetic component. The position and/or orientation can be for a k-wire to be used during the surgical procedure to guide an instrument for preparing the glenoid to receive the implant. The patient specific instrument can be made or manufactured using a variety of techniques, such as rapid prototyping techniques.

Embodiments of the invention will now be described, by way of example only, and with reference to the accompanying drawings, in which:
Figure 1 shows a plan view of an instrument according to a first embodiment of the invention;
Figure 2 shows a side view of the instrument shown in Figure 1;
Figure 3 shows a cross sectional view along line AA' of the instrument shown in Figures 1 and 2;
Figure 4 shows plan view of a variant of the first embodiment of the invention shown in Figure 1;
Figure 5 shows a plan view of an instrument according to a second embodiment of the invention;
Figure 6 shows a side view of the instrument shown in Figure 5;
Figure 7 shows a cross sectional view along line BB' of the instrument shown in Figures 5 and 6 in a first state being presented to the glenoid;
Figure 8 shows a cross sectional view along line BB' of the instrument shown in Figures 5 and 6 in a second state attached to the glenoid;
Figure 9 shows plan view of a variant of the second embodiment of the invention shown in Figure 5;
Figure 10 shows a plan view of part of an instrument according to a third embodiment of the invention;
Figure 11 shows a side view of the part of the instrument shown in Figure 10;
Figure 12 shows a plan view of further parts of the instrument shown in Figure 10;
Figure 13 shows a side view of the parts of the instrument shown in Figure 12s;
Figure 14 shows a plan view of the complete instrument according to the third embodiment of the invention;
Figure 15 shows a side view of the instrument shown in Figure 14 in use;
Figure 16 shows a cross sectional view along line CC' of the instrument shown in Figures 14 and 15;
Figure 17A shows a plan view of an example instrument;
Figure 17B shows a cross sectional view along line DD' of the instrument shown in Figure 17A;
Figure 18 shows a plan view of a further example instrument;
Figure 19 shows a side view of the instrument of Figure 18 being used to guide a drill;
Figure 20 shows a side view of the instrument of Figure 18 being used to guide a screw; and
Figure 21 shows a flow chart illustrating some of the steps of a method aspect of the invention.

Similar items in different Figures share common reference numerals unless indicated otherwise.

Although embodiments of the invention will be discussed below in the context of a shoulder arthroplasty procedure and the glenoid, it will be appreciated that the invention can also be used in hip arthroplasty procedures on the acetabulum. Some hip arthroplasty procedures involve the placement of an acetabular cup component in the acetabulum of the patient. The correct angular positioning of the cup, in terms of its version/anteversion and inclination can be important factors in the success of the hip procedure. Therefore, the patient specific instrument of the invention can be used on the acetabulum to guide tools or instruments used during preparation of the acetabulum and/or placement of the acetabular cup at the planned angular orientation relative to the patient's pelvis.

The instrument of the invention is customised to the patient on which it is to be used. The instrument is customised based on a patient's anatomy and also a patient specific surgical plan in order to address the problems surgeons experience in trying accurately to 'transfer' the pre-planned implant position by correctly placing instruments used during the arthroplasty procedure, for example to prepare the bone at the surgical site to receive the implant.

The instrument provided is customized to the individual patient's anatomy and the specific surgical plan for the patient and is in the form of a template or templates, which can be attached intra-operatively to the bony socket, e.g. glenoid or acetabulum, for example by clipping on the rim, and without using ancillary fixings, such as fixation pins or screws. The instrument is provided as a plurality of specially formed parts which can be assembled and locked together so that the instrument can be attached to the glenoid or acetabulum only at a single, unique position so that the instrument is correctly positioned to implement the surgical plan. The instrument either cannot be assembled and/or will not stay assembled if it is attempted to attach the instrument in a position other than the intended position and so the instrument can automatically provide an indication that it is being incorrectly positioned in-situ relative to the pre-operative plan.

The instrument, or templating portion, can be configured to take into account version angle corrections required for Glenoid osteoarthritis that occur for Glenoid surface rim damage.

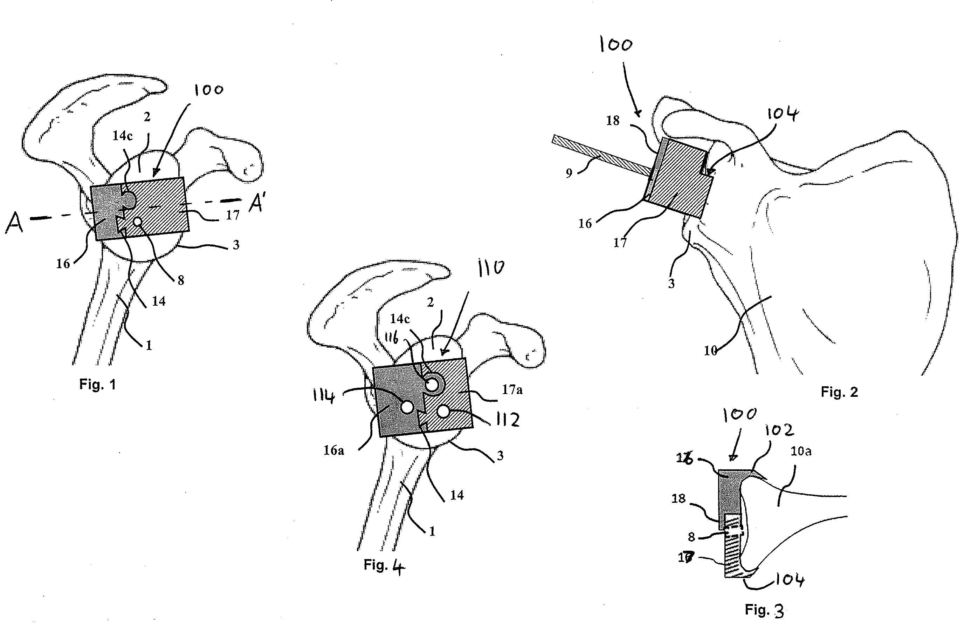

With reference to Figure 1 there is shown a plan view of a scapula (1) having a glenoid (2) to which an instrument (100) according to a first embodiment of the invention is attached in use. Figure 2 shows a side view of the instrument (100) being used to guide a k-wire (9) and Figure 3 a cross-section through the instrument (100) along line AA'. The instrument (100) has a main body formed of first (16) and second (17).parts. The second part of the body includes an aperture (8) sized to receive a k-wire (9) as illustrated in Figure 2. As illustrated in Figure 3, aperture (8) has a position relative to the glenoid and also an orientation, that is angular direction, relative to the glenoid. The instrument has been customised and preconfigured during manufacture to ensure that the instrument can be attached in a single way to the glenoid of the patient so that the position of the aperture (8) relative to the glenoid and orientation of the aperture (8) relative to the glenoid correspond to a planned position and orientation at which the k-wire should be inserted into the glenoid to guide instruments subsequently used during implantation of a glenoid implant component.

As shown in Figure 1, the first (16) and second (17) parts of the body are attached by interlocking formations (14, 14c) in the form of tabs or lugs which can engage with corresponding shaped recesses. The lugs and recesses are similar to those used in jigsaw puzzles. Further, they provide an interference fit so as to allow the two parts of the body of the instrument to be securely fastened and locked together in a releasable manner.

The instrument (100) also includes an attachment mechanism in the form of a pair of generally opposed clips (102, 104) as best seen in Figure 3. A clip portion extends from a lower part of each of the parts of the main body. Each portion of the attachment mechanism, i.e. clip (102, 104), has a shape which matches the shape of the surface rim (3) of the glenoid over the portion of the rim of the glenoid (3) to which the clip is intended to attach. The shape of the clip matches the shape of the glenoid rim both in the generally transverse direction shown in Figure 3, and also in a lateral direction extending along a portion of the periphery of the glenoid rim (3) as shown in Figure 3. That is, the clips curve in the plane illustrated in Figure 1 to match the peripheral shape of the portion of the glenoid rim. The clips also curve to match the transverse shape of the rim in the plane illustrated in Figure 3. The shape of the clip matches the shape of the rim in the transverse direction shown in Figure 3 over a substantial portion. In other embodiments, the shape of the clip can extend further into the glenoid cavity or indeed portions of the main body of the instrument may also match the shape of the inner surface of the glenoid to further improve the registration of the free parts of the instrument with the appropriate portions of the glenoid.

In use, a surgeon takes the first part of the instrument (16) and places its clip portion around the rim of the glenoid until it snugly fits. As the shape of the clip matches the shape of the rim of the glenoid by feel alone, the surgeon should be able to determine when the first portion is appropriately placed. The surgeon then also presents the clip of the second part (17) of the instrument to the rim of the glenoid which again will have a single location at which it will fit snugly against the rim of the glenoid. The surgeon can then manipulate the first and second parts of the instrument so as to engage tabs (14, 14c) in the matching recesses in the other of the parts so a to assemble and interlock the instrument as it is attached to the glenoid. If there are multiple places in which the first or second component can be attached the glenoid, then it is likely that the other of the two components will not match the local shape of the rim of the glenoid for the incorrectly positioned first piece and therefore the instrument cannot be assembled. Therefore, by customising the shape of the instrument and providing an interlock mechanism by which the separate parts of the instrument can be assembled, but only if located at a single uniquely defined position, the positioning of the instrument on the glenoid is automatically determined simply by its shape. Also, the interlocking mechanism and clips mean that no further instruments are required in order to secure the instrument to the glenoid. That is, no ancillary fittings separate to the instrument are needed, such as fixation pins or screws.

Aperture (8) provides a guide formation by which a further component or instrument used in the surgical procedure can be guided to its intended position and orientation. The guide formation (8) cannot move relative to the body of the instrument, and therefore once the instrument has been assembled in the pre-planned unique position, the position and orientation of aperture (8) is also uniquely defined. Therefore, as illustrated in Figure 2, a k-wire (9) can be inserted via guide formation aperture (8) into the bone stock (10a) behind the glenoid. The parts of the instrument can then be disassembled and removed from the glenoid, leaving the k-wire in place. The k-wire can then be used to guide other instruments, such as a cannulated reamer, or other components used in the shoulder arthroplasty procedure.

The orientation of the k-wire is pre-planned by the surgeon to take into account the glenoid plane defined by at least the most anterior, posterior and / or superior, inferior areas on the glenoid surface rim (3). The version angle of the guide aperture (8) is defined as the axial tilt of the glenoid surface which is defined by the angle between the transverse axis of the scapula and anterior-posterior face of the mid-glenoid cavity level. Inclination of the guide aperture (8) is defined as the vertical orientation of the glenoid as defined by the angle between the transverse scapula axis and the superior-inferior glenoid face. Hence, when the k-wire (9) is placed through guidance aperture (8) on to the scapula, its position is that according to the pre-planned entry point as well as pre-planned inclination and version angle orientations. A cannulated reamer, or other bone preparation instrument, can then be placed over the k-wire after the instrument (100) has been disassembled. Hence, the guided placement of the k-wire can allow subsequent instruments and components to be more accurately positioned and a more accurate reproduction of the planned version and inclination be provided.

Figure 4 shows a variation (110) of the instrument (100) shown in Figures 1, 2 and 3. Instrument (110) is similar to instrument (100) in that it comprises a main body comprising two interlocking parts (16a, 17a) and an attachment mechanism in the form of two glenoid rim shape matching clips. The two parts of the body (16a, 17a) are similarly interlockable by pairs of mating lugs and recesses (14, 14c) and the body of the instrument bears a guide formation in the form of three apertures (112, 114, 116). The three apertures (112, 114, 116) provide a guide formation for guiding a drill bit in order to allow anchor pegs to be drilled into the glenoid. Hence, this instrument operates similarly to the instrument shown in Figures 1, 2 and 3, but allows guidance of a different component used in the shoulder arthroplasty procedure to that guided by the first instrument (100). The instrument (110) is a dedicated template allowing the accurate positioning of eccentric anchor pegs. This allows the surgeon to form a more reliable bone drilled step during the procedure. The drill guide formations (112, 114, 116) correspond to the pre-planned implant size, orientation and position to better use available bone stock.

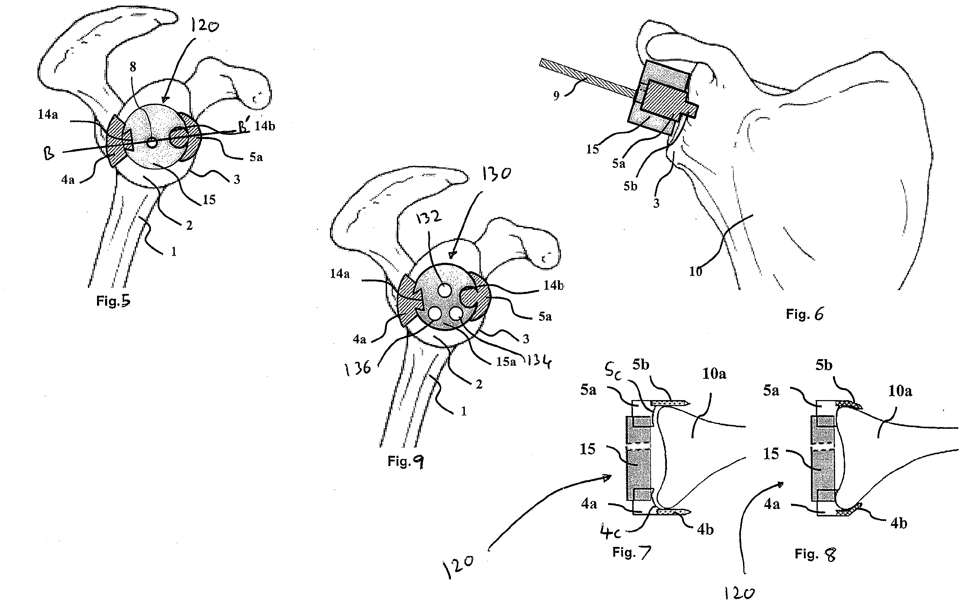

A second embodiment of an instrument (120) for use in a shoulder arthroplasty procedure is illustrated in Figures 5, 6, 7 and 8. This instrument operates generally similarly to the first instrument. However, the second embodiment (120) of the instrument of the invention has a single body part (15) bearing a guide formation in the form of aperture (8). The instrument (20) includes an attachment mechanism comprising a first part (4a) and second part (5a). The parts of the attachment mechanism (4a, 5a) include lugs (14a, 14b) which are received in correspondingly shaped recesses in the body (15) so that the parts of the instrument can be interlocked together to assemble the instrument in use. As illustrated best in Figures 7 and 8 the first (4a) and second (5a) parts of the attachment mechanism each have a two part construction. A first part (4a, 5a) bears the lugs (14a, 14b) by which the attachment mechanism interlocks with the body (15). As can be seen in Figure 7, the main portions (4a, 5a) of the attachment mechanism include at least a portion of a glenoid facing surface (4c, 5c) which is shaped to match the local shape of a portion of the glenoid rim (3). Extending from an outer end of each of the main portions (5a, 4a) are clip portions (4b, 5b) made from a shape memory material. The clip parts (4b, 5b) can be made from any suitable shaped memory material, such as various alloys or shape memory plastics. For example, the alloy provided under the name Nitinol can be used. The shape-memory material clips (4b, 5b) are used to establish attachment of the instrument on to the glenoid rim (3) by recovering to the original shape of the local portion of the rim of the glenoid as explained in greater detail with reference to Figures 7 and 8.

As can be seen in Figures 7 and 8, the main part of the attachment mechanism (4a, 5a) that engages specific locations on the medial surface rim area have surfaces (4c, 5c) shaped to match the local shape of the surface rim areas. Figure 7 shows the instrument (120) in a first state in which the instrument is being presented to the glenoid. The shape-memory material clips (4b, 5b) have not recovered their shape. In this state some fine adjustment of the positioning of the instrument is possible. However, the shapes of the main portions (4a, 5a) of the attachment mechanism and also the interlocked assembly of the attachment mechanism and body (15) mean that the instrument is automatically located generally in the correct position relative to the glenoid.

With the instrument finally positioned, by changing the temperature of the shape-memory material clips, they recover their original shape so as to clamp about the glenoid rim, as illustrated in Figure 8, so a to securely attach the instrument (120) in the pre-planned position. The shape-memory material clips (4b, 5b) have been pre-engineered so that their recovered shape matches the shape of the rim of the glenoid in both the transverse direction illustrated in Figure 8 and also around the periphery of the glenoid rim in a longitudinal direction as shown in Figure 5. Hence, any mis-positioning of the instrument will become apparent once the shape-memory clips attempt to recover their original shape resulting in either loosening or incorrect clamping of the instrument. The instrument could then be re-positioned simply by again changing the temperature of the shape-memory material clips. Assuming correct positioning, Figure 8 shows the instrument (120) form fit connected on to the glenoid surface rim (3) in the pre-planned position so that the position and orientation of guide formation (8) is also as planned. Hence, similarly to as described above, k-wire (9) can be introduced into guide aperture (8) with the pre-planned position and orientation to allow more accurate positioning of instruments and implants subsequently used in the shoulder arthroplasty procedure.

Figure 9 shows a modular aspect of the second embodiment of the invention. In this variation, instrument (130) is similar to the instrument (120) shown in Figure 5 in that the attachment mechanism is identical, but the body (15) of instrument (120) has been substituted with a different body (15a) bearing a different guide formation. As illustrated in Figure 9, body (15a) bears three apertures acting as drill guides by which a drilling step can be accomplished to locate anchor pegs. Hence, this embodiment of the invention provides a more modular system in which the same attachment mechanism is used but different bodies can be swapped in and out of the attachment mechanism to provide different templates for guiding different steps in the procedure.

Although, as described above, only a portion of the attachment mechanism is made from a shape-memory material, it will be appreciated that in other embodiments other portions or the entirety, of the attachment mechanism can be made from a shape-memory material so that some or all of the glenoid surface shape matching portions of the attachment mechanism may transition between a first state and a second state in which the glenoid engaging portion of the attachment mechanism has a shape matching that of the rim of the glenoid to which the attachment portion has been planned to be attached.

The second embodiment of the instrument (120, 130) is easier to assemble within a standard (delto-pectoral) anterior approach where the glenoid preparation is performed by a limited lateral exposure. During assembly of the instrument, the pair of attachment mechanism clips (4a, 5a) are initially placed on the glenoid rim areas and then the main body (15, 15a) is introduced from the lateral direction and manipulated to engage the lugs and apertures so as to interlock the parts of the instrument together to allow the instrument to be attached at the single pre-planned position relative to the glenoid. As the shapes of the lugs (14a, 14b) and matching apertures in the main body (15) are different for the two portions of the attachment mechanism, it is not possible to assemble the instrument in any other way than the way shown in Figure 5, e.g. it is not possible to assemble the instrument with the main body rotated by approximately 180° relative to its orientation shown in Figure 5. Hence, the form of the interlocking mechanism also contributes to uniquely defining the way in which the instrument can be assembled.

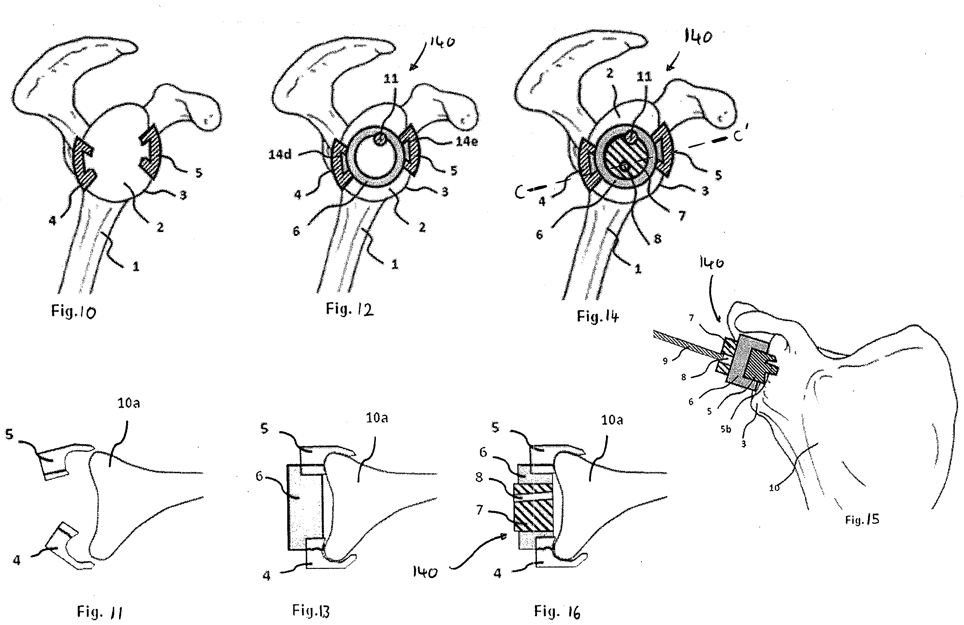

With reference to Figures 10 to 15, there is shown a third embodiment of an instrument (140) according to the invention. The instrument (140) is similar to the previously described embodiments of the invention in that it includes an attachment mechanism and body bearing a guide formation in which the parts of the instrument can be interlocked to uniquely attach the instrument to a single position on the glenoid of the patient.

As illustrated in Figures 10 and 11, the attachment mechanism includes a pair of clips (4, 5). Each clip (4, 5) has a surface portion whose shape matches the shape of the surface of the glenoid rim over the portion of the glenoid rim. The shape of the clip matches the shape of the glenoid both in a transverse direction shown in Figure 11, and in a longitudinal direction extending around the periphery of the rim as illustrated in Figure 10. Each clip (4, 5), includes a recess for accepting a lug as part of assembly and interlocking of the parts of the instrument. As illustrated in Figures 12 and 13, the body of the instrument includes a frame (6) in the form of a circular ring having a central aperture and lugs (14d, 14e) extending from generally opposed outer surface portions of the frame (6). Lugs (14d, 14e) are shaped to be received in correspondingly shaped recesses in clip portions (4, 5) of the attachment mechanism. Figure 12 shows the frame (6) attached to and interlocking with the attachment formation so as to attach the instrument to the glenoid in the pre-planned single unique position. As illustrated in Figure 14, the body of the instrument also includes a central core (7) which can be mounted within the central aperture of frame (6). Core (7) of the body includes a guide formation in the form of aperture (8) for receiving and guiding the positioning of a k-wire (9) as illustrated in Figure 15.

Instrument (140) also includes a limiter device (11) which can be used to limit the rotation of core (7) within frame (6). As illustrated in Figure 14, the limiter device includes a generally circular cross section peg member which can be received in a recess within an inner wall of frame (6). A similarly shaped recess is provided in an outer wall of core (7) so that the core (7) can be located in frame (6) in a single orientation. This therefore prevents rotation of core (7) within frame (6). However, in some circumstances, rotational fine adjustment of the core part (7) may be desirable. In such cases, peg (11) can be removed from between frame (6) and core (7) so as to allow rotation of core (7) within frame (8) so as to slightly adjust the position of guide formation (8).

In use, firstly the clips (4, 5) are positioned on the rim of the glenoid. The glenoid rim surface matching shape of the clips means that they can be snugly placed on the rim of the glenoid at single pre-defined positions. Then, as illustrated in Figure 13, the frame (16) is attached to the clips (4, 5) by lugs (14d and 14e) being received in corresponding recesses in the clips so as to interlock the parts of the instrument and attach it to the glenoid in the single redefined position. Lugs (14d, 14e) have different shapes and this precludes attachment of the frame (6) in a different orientation to that shown in Figure 12. Therefore, again, the geometry of the parts of the instrument prevent accidental assembly of the instrument in an incorrect way as the instrument can only be assembled when correctly placed in the single pre-defined position on the glenoid. Then core part (7) of the body of the instrument can be inserted in frame (6) to position guide aperture (8) so that aperture (8) can be used k-wire (9) to the pre-planned entry point and pre-planned orientation relative to the glenoid.

Similarly to the second embodiment, different core portions (7) of the instrument can be provided bearing different guide formation for guiding the positioning of different parts and components used subsequently during other steps of the shoulder arthroplasty procedure.

A particular benefit of the third embodiment is that the small clip portions (4, 5) of the attachment mechanism can easily be placed within a delto-pectoral anterior approach in which the glenoid preparation must be performed using a limited lateral exposure. Clip part (4) is shaped to mirror the posterior surface of the rim of the glenoid and clip part (5) is shaped to match at least an anterior portion of the rim surface. Each of the clip elements (4, 5) can be snugly located on the rim of the glenoid in a single unit position.

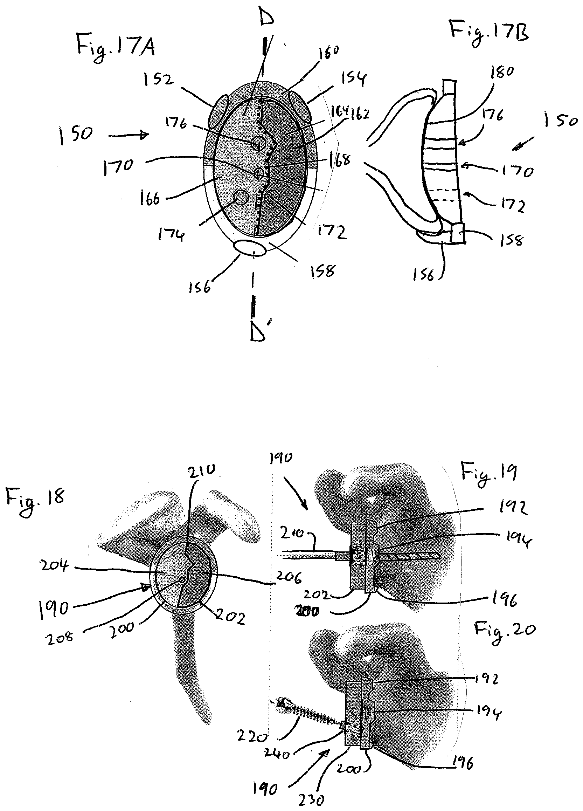

Figures 17a and 17b show an example instrument (150). Example instrument (150) is generally similar to the first through third embodiments of the invention. Instrument (150) includes an attachment mechanism comprising three clips (152, 154, 156) each of which is shaped to match the shape of corresponding portions of the rim of the glenoid. The clips (152, 154, 156) are mounted on a generally oval shaped frame comprising first (158) and second (160) parts. The first and second parts (158, 156) of the frame are attachable to form a generally oval shape with a central aperture which can receive the main body (162) of the instrument. The body of the instrument comprises first (164) and second (166) portions. Respective engaging faces of the portions of the body have mutually matching shapes corresponding to a complex shape illustrated by line (168) in Figure 17a. The complex nature of the shape of the surface by which the two portions of the body can be assembled together into a generally oval shape means that there is only one way in which the two parts of the body can be assembled together in order to fit within the frame of the instrument.

The body of the instrument bears a first guide formation (170) for receiving a k-wire in use and in the form of a circular aperture. The body also bears a second guide formation in the form of three circular apertures arranged in a generally triangular configuration (172, 174, 176). The second guide formation can be used to guide a drill for placement of pegs or other implant fixings during a step of the shoulder arthroplasty procedure different to the step in which the first guide formation (170) is used. Hence, the instrument (150) can be used to guide placement of different instruments, without having to reassemble or replace parts of the instrument.

Instrument (150) can also be used with different swappable body parts bearing different guide formations so that the different bodies can be swapped in and out of the frame while still attached to the glenoid in use.

As illustrated best in Figure 17b, other parts of the instrument have been customised to match the shape of the glenoid in order to reliably position the instrument. As shown particularly in Figure 17b, a rear glenoid facing surface (180) of the body of the instrument has also been customised so that it presents a surface shape generally matching the surface shape of the glenoid cavity over at least a portion of the area of the glenoid cavity. Hence, even if there were any mis-positioning of the frame of the instrument when being attached by clips (152, 154, 156), the body of the instrument could not then be properly received within the frame owing to a mismatch between the shape of the surface of the glenoid and the shape of the surface of the body at that position. Further, the complex interface between the two portions of the body prevent relative sliding of the portions of the body to allow some settling or readjustment of the relative positioning of the parts of the body. Therefore, again, the geometry of the instrument prevents the instrument being correctly assembled and the parts of the instrument interlocking when the instrument is attached to the glenoid at any position other than the single pre-planned position.

Figures 18, 19 and 20 show a further example instrument. The further example instrument is generally similar to the previously described embodiments. The further example instrument (190) includes an attachment mechanism in the form of a generally oval frame having four clips extending from the frame at top, bottom and left and right side positions. Parts of the top, bottom and right hand side clips (192, 194, 196) can be seen in Figure 19. The four clips are shaped to match the surface shape of the corresponding portions of the rim of the glenoid when the instrument is placed on the glenoid at the preplanned position. The frame portion (200) of the attachment mechanism is made of an elastic material so that the clip portions are biased toward the rim of the glenoid so as to securely attach the instrument to the glenoid in use. Instrument (190) includes a body (202) having first (204) and second (206) parts. The body (202) includes a guide formation in the form of a circular aperture (208) for guiding a component, such as a k-wire or drill bit, during the shoulder arthroplasty procedure. The body (202) has a generally oval shape and the two parts (204, 206) have faces with matching complex shapes (210). In this way, parts (204, 206) can only be assembled together into a generally oval shape when correctly aligned. The main body (202) can then be received and retained by a friction or interference fit within frame (190) which pulls the interlocking parts of the instrument together.

Figure 19 shows a side view of instrument (190) assembled on a glenoid in use and guiding the drilling of a hole using a drill bit (210) passing through guide aperture (208).

Figure 20 shows a side view of instrument (190) being used to guide placement of a screw (220). In this variant, the body (202) of the instrument has been replaced with a second body (230) having a similar general shape but with a different guide formation (234) suitable for receiving and guiding placement and orientation of screw (220).

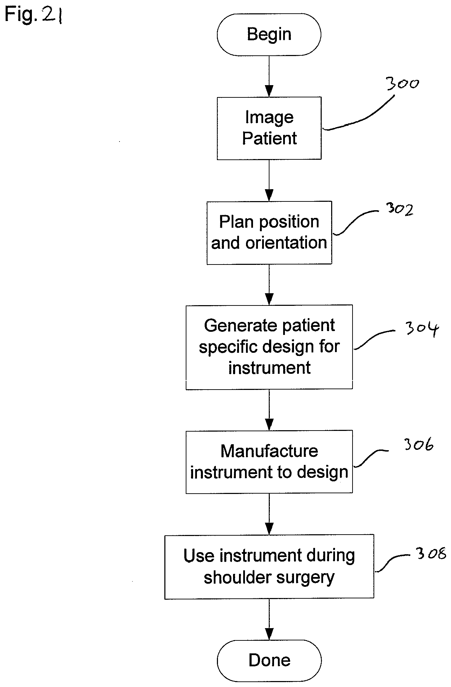

Figure 21 shows a flow chart illustrating various steps of a method of the invention. The various steps of the method illustrated in Figure 21 include both the manufacture and use of the customised patient specific implant as described previously. At an initial step (300), the patient is imaged to capture 3D image data, including at least a 3D image of the shoulder and particularly the glenoid of the patient. Various imaging modalities can be used. In a particular embodiment, a 3D CT image of the patient's shoulder is captured and data representing the 3D image of the patient's shoulder is stored.

Then at step 302, the surgeon using planning software to plan the shoulder arthroplasty procedure. This can involve planning the size of the implant components, their position and orientation relative to the patient's bones. In particular, the size, position and orientation of the glenoid component of a total shoulder arthroplasty can be planned. The software uses the 3D image of the patient's shoulder and the surgeon can select a suitably sized glenoid component and determine its intended implant position and orientation in order to achieve the surgeon's intention. By planning the intended position and orientation of the glenoid component, the software has access to information determining the ideal position and orientation for the placement of instrumentation used during the surgical procedure. Hence, the software can determine the optimum position and orientation by which the k-wire should be introduced into the glenoid in order to guide the positioning and orientation of other instrumentation used during the procedure.

Then at step 304, a patient specific instrument is designed using the planning information derived from the planning software at step 302 and also information from the image of the patient which defines the shape of the glenoid of the patient. Hence, a software application can determine the appropriate shape for the attachment formation parts of the instrument, and any of the other parts of the instrument, whose shape is designed to be patient specific so as to match the local shape of the portion of the glenoid to which the instrument will attach a single pre-planned position. Then, for that single planned position of attachment of the instrument, the position and orientation of the guide formations of the instrument can be determined as the intended position of the instrument relative to the glenoid has now been fixed and also the position and orientation of the k-wire relative to the glenoid has previously been planned. Therefore, at step 304, the design of the instrument can be generated.

The design of the instrument is then used to manufacture an instrument according to the design and customised to the patient's specific anatomy and also the patient's specific planned surgical procedure. A variety of techniques can be used to manufacture the instrument. For example, rapid prototyping type techniques can be used. The instrument itself can be made from a number of suitable bio-compatible materials, such as metals, alloys, plastics, polymers or similar.

Once the instrument has been made to the design at step 306, at step 308 the instrument can be used during the shoulder arthroplasty procedure as generally described above. As will be understood from the preceding description, the instrument has been customised to be attachable to the patient's glenoid in a single unique position. Also, the guide formation has been placed and oriented in the instrument so that once the instrument is attached to the patient's glenoid at that single position, the guide formation will automatically be registered with the intended point of entry and angle or orientation relative to the glenoid as previously planned. Hence, the subsequent reliability of the positioning of the implant as pre-planned is improved.

After the instrument has served its purpose or purposes during the surgical procedure, it can easily be diss-assembled *in situ* simply by undoing the interlocking parts, or changing its temperature for the shape memory material embodiment, allowing it to be easily removed to provide better access to the surgical site and without the need to undo ancillary fixings or fasteners. Hence, the ease and speed of use of the instrument is enhanced.

Further, as discussed above, no further ancillary components are required in order to attach the instrument to the glenoid. Rather, owing to the interlocking nature of the separate parts of the instrument, mere assembly of the interlocking parts of the instrument causes its attachment to the glenoid at its intended position. Also, trauma or damage to the surgical site is reduced as no ancillary fixings or fasteners need to be used As will be apparent from the above discussion, there are a wide number of variations and modifications possible to the instrument of the invention. The invention is not limited to the specific embodiments described above. Rather, various modifications and changes are envisaged. As discussed above, the invention can also be used to provide a patient specific instrument for use on the acetabulum. The various features of the invention described with reference to one embodiment may be swapped, mixed with, added to or removed, from the other described embodiments of the invention. Therefore, the scope of the invention is defined and limited only by the appended claims.

## Claims

1. A patient specific instrument (100, 110, 120, 130, 140) for use in a ball and socket joint arthroplasty procedure to be carried out on a patient, comprising:
a body (15, 15a, 16, 17) bearing at least one guide formation (8, 112, 114, 116, 132, 134, 136) configured for guiding a component to be used in the ball and socket joint arthroplasty procedure; and
an attachment mechanism for attaching the instrument to the socket of the patient, wherein the instrument has a plurality of parts, the attachment mechanism has at least first (4, 4b, 102) and second (5, 5b, 104) parts configured during manufacture using information from an image of the socket of a patient in order to engage the rim of the socket of the patient, each part being shaped, or adapted to transform to a shape, to match the shape of a respective portion of the rim of the socket; **characterized in that** the instrument further comprises a locking mechanism comprising interlocking formations (14, 14c, 14a, 14b,14d, 14e) by which the body and attachment mechanism are adapted to be assembled in a unique configuration into the patient specific instrument in which the patient specific instrument is configured to be attached to the socket of the patient only at a single pre-planned position.

2. The patient specific instrument (110, 130) as claimed in claim 1, wherein the body includes a plurality of guide formations (112, 114, 116, 132, 134, 136) configured for guiding different components.

3. The patient specific instrument as claimed in claim 1 or 2, wherein the attachment mechanism includes at least three parts each configured during manufacture using information from an image of the socket of a patient in order to engage the rim of the socket, each part being shaped, or adapted to transform to a shape, to match the shape of a respective portion of the rim of the socket.

4. The patient specific instrument as claimed in any of claims 1 to 3, wherein the parts of the attachment mechanism are separate parts.

5. The patient specific instrument as claimed in claim 4, wherein the parts of the attachment mechanism are clips.

6. The patient specific instrument as claimed in any of claims 1 to 3, wherein the parts of the attachment mechanism are parts of the same structure.

7. The patient specific instrument (100, 140) as claimed in any preceding claim, wherein the body comprises a plurality of parts.

8. The patient specific instrument (100) as claimed in claim 7 wherein each part of the body includes a respective portion of the attachment mechanism and wherein interlocking formations (14, 14c) of the parts of the body provide the locking mechanism.

9. The patient specific instrument (120, 140) as claimed in any of claims 1 to 5, wherein the locking mechanism is provided by interlocking formations on the body (15) and the parts (4, 4a, 4b, 5) of the attachment mechanism.

10. The patient specific instrument (120) as claimed in any preceding claim, wherein the attachment mechanism is partially (4b, 5b) or wholly a shape memory material adapted to transition between a first state in which the instrument is presentable to the socket and a second state in which the attachment mechanism is configured to attach the instrument to the socket only at the single pre-planned position.

11. The patient specific instrument as claimed in any preceding claim, wherein the attachment mechanism is at least partially a shape memory material and the shape memory material allows each of the first and second parts to transform to a shape to match the shape of a respective rim portion of the rim of the socket.

12. The patient specific instrument (140) as claimed in any of claims 1 to 4, wherein the body comprises a frame (6) and a core (7) which is receivable in the frame and wherein the core includes the at least one guide formation (8).

13. The patient specific instrument (140) as claimed in claim 12, wherein the locking mechanism is provided by interlocking formations (14d, 14e) on the frame (6) and the parts (4, 5) of the attachment mechanism.

14. The patient specific instrument (140) as claimed in claim 13, further comprising a limiter (11) allowing the core (7) to be received in the frame (6) in a single position.

15. The patient specific instrument as claimed in claim 12, wherein the frame is made wholly or at least partially from an elastic material.

16. A method for producing a patient specific instrument according to any of claim 1 to 15, the method comprising:
capturing (300) image data representing a three dimensional image of at least the socket of the patient;
planning (302) a position and/or orientation to be used in the ball and socket arthroplasty procedure using the captured image data;
designing (304) a patient specific instrument; and
making (306) a patient specific instrument according to the design.

## Patentansprüche

1. Patientenspezifisches Instrument (100, 110, 120, 130, 140) zur Verwendung in einem Kugelgelenk-Arthroplastikverfahren, das an einem Patienten durchzuführen ist, wobei das Instrument umfasst:
einen Körper (15, 15a, 16, 17), der mindestens eine Führungsformation (8, 112, 114, 116, 132, 134, 136) trägt, der konfiguriert ist zum Führen einer Komponente, die bei dem Kugelgelenk-Arthroplastikverfahren zu verwenden ist; und
einen Anbringungsmechanismus zum Anbringen des Instruments an der Pfanne des Patienten, wobei das Instrument eine Mehrzahl von Teilen aufweist, wobei der Anbringungsmechanismus zumindest erste (4, 4b, 102) und zweite (5, 5b, 104) Teile aufweist, die während der Herstellung konfiguriert werden, und zwar unter Verwendung von Informationen aus einem Bild der Pfanne des Patienten, um in den Rand der Pfanne des Patienten einzugreifen, wobei jedes Teil geformt oder angepasst ist, sich in eine Form umzuwandeln, um zu der Form eines jeweiligen Abschnitts des Rands der Pfanne zu passen;
**dadurch gekennzeichnet, dass** das Instrument ferner einen Verriegelungsmechanismus umfasst, der Verriegelungsformationen (14, 14c, 14a, 14b, 14d, 14e) umfasst, durch die der Körper und der Anbringungsmechanismus angepasst sind, in einer einzigartigen Konfiguration in das patientenspezifische Instrument montiert zu werden, in der das patientenspezifische Instrument konfiguriert ist, nur an einer einzigen vorgeplanten Position an der Pfanne des Patienten angebracht zu werden.

2. Patientenspezifisches Instrument (110, 130) nach Anspruch 1, wobei der Körper eine Mehrzahl von Führungsformationen (112, 114, 116, 132, 134, 136) aufweist, die zum Führen unterschiedlicher Komponenten konfiguriert sind.

3. Patientenspezifisches Instrument nach Anspruch 1 oder 2, wobei der Anbringungsmechanismus zumindest drei Teile enthält, von denen jeder während der Herstellung konfiguriert wird, und zwar unter Verwendung von Informationen aus einem Bild der Pfanne eines Patienten, um in den Rand der Pfanne einzugreifen, wobei jeder Teil geformt oder angepasst ist, sich in eine Form umzuwandeln, um zu der Form eines jeweiligen Abschnitts des Rands der Pfanne zu passen.

4. Patientenspezifisches Instrument nach einem der Ansprüche 1 bis 3, wobei die Teile des Anbringungsmechanismus separate Teile sind.

5. Patientenspezifisches Instrument nach Anspruch 4, wobei die Teile des Anbringungsmechanismus Clips sind.

6. Patientenspezifisches Instrument nach einem der Ansprüche 1 bis 3, wobei die Teile des Anbringungsmechanismus Teile der gleichen Struktur sind.

7. Patientenspezifisches Instrument (100, 140) nach einem der vorhergehenden Ansprüche, wobei der Körper eine Mehrzahl von Teilen umfasst.

8. Patientenspezifisches Instrument (100) nach Anspruch 7, wobei jeder Teil des Körpers einen jeweiligen Abschnitt des Anbringungsmechanismus enthält und wobei ineinander verriegelnde Formationen (14, 14c) der Teile des Körpers den Verriegelungsmechanismus bereitstellen.

9. Patientenspezifisches Instrument (120, 140) nach einem der Ansprüche 1 bis 5, wobei der Verriegelungsmechanismus durch ineinander verriegelnde Formationen an dem Körper (15) und die Teile (4, 4a, 4b, 5) des Anbringungsmechanismus bereitgestellt ist.

10. Patientenspezifisches Instrument (120) nach einem der vorhergehenden Ansprüche, wobei der Anbringungsmechanismus teilweise (4b, 5b) oder vollständig ein Formgedächtnismaterial ist, das angepasst ist, zwischen einem ersten Zustand, in dem das Instrument der Pfanne präsentierbar ist, und einem zweiten Zustand überzugehen, in dem der Anbringungsmechanismus konfiguriert ist, das Instrument an der Pfanne nur an der einzigen vorgeplanten Position anzubringen.

11. Patientenspezifisches Instrument nach einem der vorhergehenden Ansprüche, wobei der Anbringungsmechanismus zumindest teilweise ein Formgedächtnismaterial ist und das Formgedächtnismaterial es jedem des ersten und des zweiten Teils erlaubt, sich in eine Form umzuwandeln, um mit der Form eines jeweiligen Randabschnitts des Randes der Pfanne übereinzustimmen.

12. Patientenspezifisches Instrument (140) nach einem der Ansprüche 1 bis 4, wobei der Körper einen Rahmen (6) und einen Kern (7) umfasst, der in dem Rahmen aufnehmbar ist, und wobei der Kern die zumindest eine Führungsformation (8) enthält.

13. Patientenspezifisches Instrument (140) nach Anspruch 12, wobei der Verriegelungsmechanismus durch ineinander verriegelnde Formationen (14d, 14e) an dem Rahmen (6) und den Teilen (4, 5) des Anbringungsmechanismus bereitgestellt ist.

14. Patientenspezifisches Instrument (140) nach Anspruch 13, ferner umfassend einen Begrenzer (11), der es dem Kern (7) erlaubt, in dem Rahmen (6) in einer einzelnen bzw. einzigen Position aufgenommen zu werden.

15. Patientenspezifisches Instrument nach Anspruch 12, wobei der Rahmen vollständig oder zumindest teilweise aus einem elastischen Material besteht.

16. Verfahren zum Herstellen eines patientenspezifischen Instrumentes nach einem der Ansprüche 1 bis 15, wobei das Verfahren umfasst:
Erfassen (300) von Bilddaten, die ein dreidimensionales Bild von zumindest der Pfanne des Patienten darstellen;
Planen (302) einer Position und/oder Ausrichtung, die in dem Kugelgelenk-Arthroplastikverfahren zu verwenden ist, unter Verwendung der aufgenommenen Bilddaten;
Entwerfen (304) eines patientenspezifischen Instruments; und
Erstellen (306) eines patientenspezifischen Instruments gemäß dem Design.

## Revendications

1. Instrument spécifique au patient (100, 110, 120, 130, 140) pour utilisation dans une procédure d'arthroplastie à énarthrose à réaliser sur un patient, comprenant :
un corps (15, 15a, 16, 17) portant au moins une formation de guidage (8, 112, 114, 116, 132, 134, 136) configurée pour guider un composant à utiliser dans la procédure d'arthroplastie à énarthrose ; et
un mécanisme de fixation pour fixer l'instrument à l'emboîture du patient, dans lequel l'instrument comporte une pluralité de parties, le mécanisme de fixation comporte au moins des première (4, 4b, 102) et seconde (5, 5b, 104) parties configurées durant la fabrication en utilisant des informations provenant d'une image de l'emboîture d'un patient afin d'entrer en prise avec le rebord de l'emboîture du patient, chaque partie étant façonnée, ou adaptée pour se transformer à une forme, pour correspondre à la forme d'une portion respective du rebord de l'emboîture ;
**caractérisé en ce que** l'instrument comprend en outre un mécanisme de verrouillage comprenant des formations d'interverrouillage (14, 14c, 14a, 14b, 14d, 14e) par lesquelles le corps et le mécanisme de fixation sont adaptés pour être assemblés dans une unique configuration dans l'instrument spécifique au patient dans lequel l'instrument spécifique au patient est configuré pour être fixé à l'emboîture du patient seulement dans une position pré-planifiée unique.

2. Instrument spécifique au patient (110, 130) selon la revendication 1, dans lequel le corps inclut une pluralité de formations de guidage (112, 114, 116, 132, 134, 136) configurées pour guider différents composants.

3. Instrument spécifique au patient selon la revendication 1 ou 2, dans lequel le mécanisme de fixation inclut au moins trois parties configurées chacune durant la fabrication en utilisant des informations provenant d'une image de l'emboîture d'un patient afin d'entrer en prise avec le rebord de l'emboîture, chaque partie étant façonnée, ou adaptée pour se transformer à une forme, pour correspondre à la forme d'une portion respective du rebord de l'emboîture.

4. Instrument spécifique au patient selon l'une quelconque des revendications 1 à 3, dans lequel les parties du mécanisme de fixation sont des parties séparées.

5. Instrument spécifique au patient selon la revendication 4, dans lequel les parties du mécanisme de fixation sont des clips.

6. Instrument spécifique au patient selon l'une quelconque des revendications 1 à 3, dans lequel les parties du mécanisme de fixation sont des parties de la même structure.

7. Instrument spécifique au patient (100, 140) selon une quelconque revendication précédente, dans lequel le corps comprend une pluralité de parties.

8. Instrument spécifique au patient (100) selon la revendication 7 dans lequel chaque partie du corps inclut une portion respective du mécanisme de fixation et dans lequel des formations d'interverrouillage (14, 14c) des parties du corps fournissent le mécanisme de verrouillage.

9. Instrument spécifique au patient (120, 140) selon l'une quelconque des revendications 1 à 5, dans lequel le mécanisme de verrouillage est fourni par des formations d'interverrouillage sur le corps (15) et les parties (4, 4a, 4b, 5) du mécanisme de fixation.

10. Instrument spécifique au patient (120) selon une quelconque revendication précédente, dans lequel le mécanisme de verrouillage est partiellement (4b, 5b) ou entièrement un matériau à mémoire de forme adapté pour passer entre un premier état dans lequel l'instrument peut être présenté à l'emboîture et un second état dans lequel le mécanisme de fixation est configuré pour fixer l'instrument à l'emboîture seulement dans la position pré-planifiée unique.

11. Instrument spécifique au patient selon une quelconque revendication précédente, dans lequel le mécanisme de fixation est au moins partiellement un matériau à mémoire de forme et le matériau à mémoire de forme permet à chacune des première et seconde parties de se transformer à une forme pour correspondre à la forme d'une portion de rebord respective du rebord de l'emboîture.

12. Instrument spécifique au patient (140) selon l'une quelconque des revendications 1 à 4, dans lequel le corps comprend un cadre (6) et un noyau (7) qui peut être reçu dans le cadre et dans lequel le noyau inclut l'au moins une formation de guidage (8).

13. Instrument spécifique au patient (140) selon la revendication 12, dans lequel le mécanisme de verrouillage est fourni par des formations d'interverrouillage (14d, 14e) sur le cadre (6) et les parties (4, 5) du mécanisme de fixation.

14. Instrument spécifique au patient (140) selon la revendication 13, comprenant en outre un limiteur (11) permettant au noyau (7) d'être reçu dans le cadre (6) dans une seule position.

15. Instrument spécifique au patient selon la revendication 12, dans lequel le cadre est composé entièrement ou au moins partiellement à partir d'un matériau élastique.

16. Procédé de production d'un instrument spécifique au patient selon l'une quelconque des revendications 1 à 15, le procédé comprenant :
la capture (300) de données d'image représentant une image en trois dimensions d'au moins l'emboîture du patient ;
la planification (302) d'une position et/ou orientation à utiliser dans la procédure d'arthroplastie à énarthrose en utilisant les données d'image capturées ;
la conception (304) d'un instrument spécifique au patient ; et
la réalisation (306) d'un instrument spécifique au patient selon la conception.
